# EUROPÄISCHE PATENTSCHRIFT

(11) **EP 1 062 971 B1**
(45) Veröffentlichungstag und Bekanntmachung des Hinweises auf die Patenterteilung: **02.02.2005**
(21) Anmeldenummer: 00250203.7
(22) Anmeldetag: 23.06.2000
(51) Int. Cl.: A61N 1/05

(54) **Katheter mit distaler Schleuse für einen Draht**
Catheter with distal wire port
Cathéter muni d'un port distal pour un fil

(30) Priorität: 25.06.1999 DE 19930266
(43) Veröffentlichungstag der Anmeldung: 27.12.2000
(73) Patentinhaber: BIOTRONIK Mess- und Therapiegeräte GmbH & Co Ingenieurbüro Berlin, 12359 Berlin (DE)
(72) Erfinder: Witte, Joachim, 10409 Berlin (DE)
(74) Vertreter: Eisenführ, Speiser & Partner

(56) Entgegenhaltungen:
- EP-A- 0 773 037
- EP-A- 0 778 043
- WO-A-98/02201
- WO-A-99/55412
- US-A- 4 103 690
- US-A- 5 871 530

## Beschreibung

Die Erfindung bezieht sich auf einen Katheter zum Einführen in Blutgefäße mit einem Lumen und darin angeordneten Führungsmitteln.

Unter Katheter wird im Sinne dieser Beschreibung beispielsweise auch eine Elektrode samt Elektrodenleitung für Herzschrittmacher oder Defibrillatoren verstanden. Es ist eine große Anzahl verschiedener Katheter der eingangs genannten Art bekannt, die mit verschiedenen Führungsmitteln ausgestattet sind, um einen Katheter durch Blutgefäße hindurch an einen gewünschten Ort zu dirigieren. Derartige Katheter bzw. Elektroden sind beispielsweise aus der EP 0 667 126, aus der US 5,423,884, der US 5,376,109 oder der DE 35 07 119 bekannt. In US-A-4 103 690 ist ein Kaheter gemäß dem oberbegriff von Anspruch 1 offenbart.

Es hat sich herausgestellt, daß bekannte Katheter, insbesondere bekannte Herzschrittmacherelektroden, kaum geeignet sind, um beispielsweise in den Koronarsinus eines Herzens eingeführt zu werden. Es besteht jedoch der Wunsch, insbesondere eine Herzschrittmacherelektrode auch in den Koronarsinus oder ähnlich schwer zugängliche Blutgefäße einführen zu können.
Ziel der Erfindung ist es daher, einen zum Einführen in den Koronarsinus oder andere schwer zugängliche Blutgefäße geeigneten Katheter, insbesondere eine Herzschrittmacherelektrodenleitung, anzugeben.

Dieses Ziel wird erfindungsgemäß mit einem Katheter der eingangs genannten Art erreicht, bei dem die Führungsmittel einen in Längsrichtung des Katheters verschiebbaren vorgebogenen Draht umfassen und bei dem der Katheter an seinem distalen Ende eine derartige Schleuse aufweist, daß der vorgebogene Draht durch die Schleuse hindurch am distalen Ende aus dem Katheter austreten kann, wobei die schleuse eine von dem vorgebogenen Draht zu durchstoßende Membran umfasst.

Der Erfindung liegt der Gedanke zugrunde, den vorgebogenen Draht als Sonde zu verwenden, die über die Katheterspitze hinaus vorgeschoben werden kann und aufgrund der Vorbiegung des Drahtes in eine von der Geradeaus-Richtung des Katheters abweisende Richtung weist. Auf diese Weise ist es möglich, mit dem vorgebogenen Draht in eine Abzweigung der Blutgefäße vorzudringen und den dann von dem vorgebogenen Draht geführten Katheter nachzuschieben. Dabei kann der vorgebogene Draht im gleichen Maße wieder in den Katheter zurückgezogen werden, wie der Katheter vorgeschoben wird, so daß die Spitze des vorgebogenen Drahtes keine Relativbewegung gegenüber dem Blutgefäß durchzuführen braucht, wenn der Katheter nachgeschoben wird. Eine Schleuse an der Katheterspitze ermöglicht es, den vorgebogenen Draht auszuschieben, ohne daß nennenswert Körperflüssigkeit in den Katheter eindringt.

Der vorgebogene Draht ist vorzugsweise ein Federstahldraht. Er ist zumindest im Bereich seines distalen Endes helixförmig oder spiralförmig vorgebogen. Durch diese Art des Vorbiegens können verschiedene Ausrichtungen des Drahtes bewirkt werden, je nachdem, wie weit der Draht über die Katheterspitze hinaus vorgeschoben wird. Federstahldraht ist deshalb geeignet, weil Federstahl vorgebogen und dann die Federwirkung des Federstahls unter Einfluß entsprechender Rückstellkräfte wieder ausgerichtet werden kann, wobei der Federstahldraht beim Nachlassen dieser rückstellenden Kräfte wieder seine vorgebogene Form annimmt.

Vorzugsweise umfassen die Führungsmittel eine Führungshülse für den vorgebogenen Draht, die derart gestaltet ist, daß sie den vorgebogenen Draht innerhalb des Katheters in gestrecktem, vorgespanntem Zustand hält. Die Führungshülse, beispielsweise ein Röhrchen, in das der vorgebogene Draht eingeschoben ist, übt in diesem Falle die zum Strecken des Federstahldrahtes erforderlichen Rückstellkräfte aus. Wird der Federstahldraht aus dem Röhrchen vorgeschoben, wirken diese von dem Röhrchen auf den Federstahldraht ausgeübten Rückstellkräfte nicht mehr, so daß sich der Federstahldraht entsprechend seiner Vorbiegung krümmt, sobald er aus der Führungshülse austritt.

Der vorgebogene Draht ist dabei vorzugweise relativ zum übrigen Katheter um seine Längsachse drehbar ausgeführt und steht mit am proximalen Ende des Katheters angeordneten Betätigungsmitteln zum Drehen des vorgebogenen Drahtes in Verbindung. Ist der vorgebogene Draht beispielsweise spiralförmig vorgebogen, kann mit Hilfe der Betätigungsmittel bestimmt werden, in welche radiale Richtung sich der vorgebogene Draht beim Vorschieben über die Katheterspitze hinaus ausrichtet.

Vorzugsweise besitzt der Katheter einen rohrartig ausgebildeten, als Führungshülse dienenden Mandrin mit einem inneren Kanal, in dem der vorgebogene Draht geführt ist. Damit hält der Mandrin den Führungsdraht in Längsrichtung des Katheters ausgerichtet. Auch der Mandrin ist vorzugsweise im Katheter längsverschiebbar und/oder um seine Längsachse drehbar angeordnet und steht mit Betätigungsmitteln zum Längsverschieben und/oder Drehen des Mandrins in Verbindung. Mit den Betätigungsmitteln zum Verdrehen des Mandrins kann auch der in dem Mandrin geführte vorgebogene Draht gleich mitgedreht werden, so daß über die Drehung des Mandrins die Ausrichtung des vorgebogenen Drahtes bestimmt werden kann. Falls der Mandrin beispielsweise als Stilett zum Einschrauben einer Einschraubspitze für eine Elektrodenleitung ausgebildet ist, können Mandrin und vorgebogener Draht auch unabhängig voneinander drehbar ausgebildet sein.

Die Schleuse umfaßt eine von dem vorgebogenen Draht zu durchstoßende Membran. Auf diese Weise kann die Katheterspitze durch die Membran solange vollkommen geschlossen bleiben, bis der vorgebogene Draht die Membran durchstößt und vorne aus der Katheterspitze austritt. Wenn die Membran aus elastischem Material beispielsweise aus Silikon besteht, schließt sie sich wieder, wenn der vorgebogene Draht wieder in den Katheter zurückgezogen wird.

Die Schleuse weist auf ihrer dem Katheterinneren zugewandten Seite vorzugsweise eine Eintrittsöffnung für den vorgebogenen Draht auf, die trichterförmig gestaltet ist. Dies erleichert das Einfädeln der Spitze des vorgebogenen Drahtes in die Schleuse.

Weiterhin weist die Schleuse vorzugsweise eine Austrittsöffnung in der Außenseite des Katheters auf, die sich in der Mitte einer gerundeten Katheterspitze am distalen Ende des Katheters befindet. Durch die zentrale Anordnung der Austrittsöffnung auf der Katheterspitze ist die Ausrichtung des Katheters selbst beim Ausfahren des vorgebogenen Drahtes irrelevant, der Katheter verhält sich in jeder Drehwinkellage gleichartig. Die gerundete Katheterspitze trägt dazu bei, daß der Katheter dem vorgebogenen Draht beim Nachschieben leicht auch in schwer zugängliche, seitliche Blutgefäße folgen kann.

Der vorgebogene Draht ist vorzugsweise mit Gold beschichtet, so daß er beispielsweise mit einem Röntgengerät geortet werden kann, so daß der vorgebogene Draht beim Einsetzen des Katheters gezielt gesteuert werden kann.

Der Katheter ist vorzugsweise als Herzschrittmacher- oder Defibrillatorelektrode ausgebildet.

Die Erfindung soll nun anhand von Ausführungsbeispielen mit Hilfe der Figuren erläutert werden. Die Figuren zeigen:
- Figur 1: Einen Katheter mit vorgebogenem Führungsdraht in schematischer Außenansicht;
- Figur 2: Einen Schnitt durch das distale Ende eines Katheters wie demjenigen aus Figur 1 und
- Figur 3: Den Schnitt aus Figur 2 bei vorgeschobenem vorgebogenem Draht.

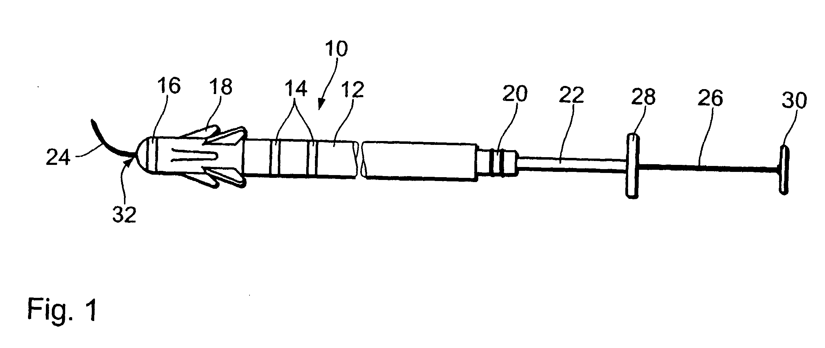

Der in Figur 1 abgebildete Katheter 10 ist als Herzschrittmacherelektrode mit einer Elektrodenleitung 12 ausgebildet, an deren distalem Ende Ringelektroden 14 und eine Tipelektrode 16 angeordnet sind. Außerdem sind am distalen Ende der Elektrodenleitung 12 Tines 18 vorgesehen, die als Abstandshalter bzw. Verankerung des Elektrodenkopfes im Myokard eines Herzens dienen können. Die Elektroden 14 und 16 sind über einen in der Elektrodenleitung 12 angeordneten, gewendelten elektrischen Leiter mit einer Steckverbindung 20 verbunden, die dazu dient, den Katheter 10 elektrisch in ein Stimulationsgerät wie einen Herzschrittmacher oder einen Defibrillator anzuschließen.

Außerdem verlaufen in der Elektrodenleitung 12 ein Mandrin 22, der rohrartig mit einem im Inneren des Mandrin 22 verlaufenden Kanal ausgebildet ist. In dem Kanal verläuft ein an seinem distalen Ende 24 vorgebogener Draht 26. Sowohl der Mandrin 22 als auch der vorgebogene Draht 26 sind an ihren proximalen Enden jeweils mit in Figur 1 schematisch dargestellten Betätigungsmitteln 28 bzw. 30 verbunden, mit denen sowohl der Mandrin 22 als auch der vorgebogene Draht 26 relativ zueinander und zu der Elektrodenleitung 12 in deren Längsrichtung verschoben und um deren Längsachse gedreht werden können.

Bereits Figur 1 ist zu entnehmen, daß der vorgebogene Draht 26 mit seinem distalen Ende 24 über die Spitze 32 des Katheters 10 hinaus vorgeschoben werden kann. Da der vorgebogene Draht 26 an seinem distalen Ende 24 vorgebogen ist, krümmt er sich beim Austritt aus der Katheterspitze 32 in die vorgebogene Richtung. Diese ist eine andere, als die verlängerte Längsachse des Katheters 10 im Bereich seines distalen Endes.

Beim Einsetzen des Katheters 10 kann dieser bis in die Nähe beispielsweise einer Blutgefäßverzweigung vorgeschoben werden. Bis dahin kann der vorgebogene Draht 26 vollständig in den Katheter 10 zurückgezogen bleiben, so daß der Katheter 10 an seiner Spitze 32 endet. In der Nähe der Blutgefäßverzweigung kann dann der vorgebogene Draht über die Katheterspitze 32 hinaus vorgeschoben werden und krümmt sich dabei in der dargestellten Weise seitlich. Durch Drehen des vorgebogenen Drahtes 26 alleine oder gemeinsam mit dem Mandrin 22 kann die ideale Richtung, in die der vorgebogene Draht 26 von der gedachten Verlängerung des distalen Endes des Katheters 10 abweicht, gewählt werden, so daß das distale Ende 24 des vorgebogenen Drahtes 26 exakt in ein Abzweigungsblutgefäß eingeführt werden kann. Um diesen Vorgang außerhalb eines zu behandelnden Körpers optisch verfolgen zu können, ist der vorgebogene Draht im Bereich seines distalen Endes 24 mit Gold beschichtet. Diese Beschichtung ist körperverträglich und macht den vorgebogenen Draht 26 mittels eines Röntgengerätes sichtbar. Nachdem der vorgebogene Draht 26 mit seinem distalen Ende 24 weit genug in ein abzweigendes Blutgefäß wie beispielsweise dem Koronarsinus eines Herzens vorgeschoben wurde, kann der Katheter 10 unter gleichzeitigem Zurückziehen des vorgebogenen Drahtes 26 in den Katheter 10 nachgeschoben werden. Auf diese Weise ist es möglich, daß der vorgebogene Draht 26 beim Nachschieben des Katheters 10 bezüglich des Blutgefäßes keine Relativbewegung durchführt, die zu Verletzungen führen könnte.

Der in Figur dargestellte Längsschnitt durch das distale Ende des Katheters 10 zeigt dieses distale Ende, bevor der vorgebogene Draht 26 über die Spitze des Katheters 32 hinausgeschoben ist. Zu erkennen sind Ringelektroden 14 sowie eine gewendelte elektrische Leitung 34, die zur Übertragung elektrischer Signale von und zu den Elektroden 14 dient. Innerhalb der gewendelten Elektrodenleitung 34 verläuft der rohrartig ausgebildete Mandrin 22, in dessen innerem Kanal 36 der vorgebogene Draht 26 geführt ist. Durch den Mandrin 36 wird der vorgebogene Draht 26 gestreckt, so daß er Inneren des Katheters 10 im wesentlichen gerade verläuft.

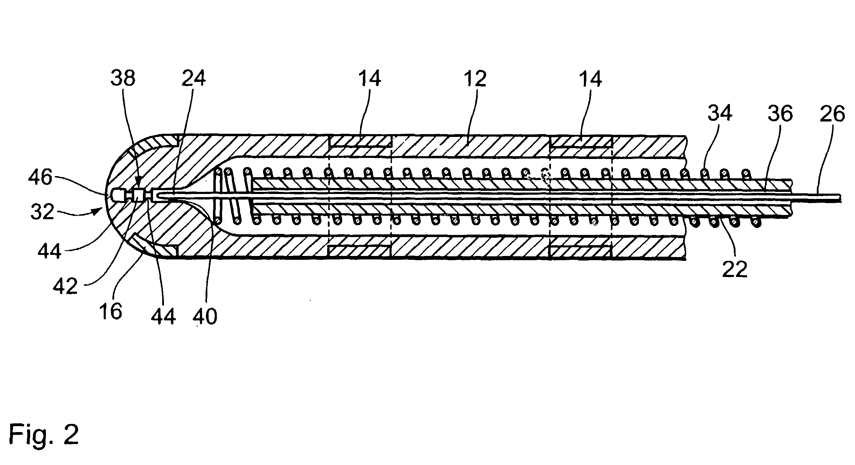

Wie Figur 2 zu entnehmen ist, weist der Katheter 10 an seinem distalen Ende eine Schleuse 38 auf, durch die der vorgebogene Draht 26 hindurchgeschoben werden kann. Die Schleuse 38 besitzt an ihrer dem Katheterinneren zugewandten Seite eine Eintrittsöffnung 40, die trichterförmig gestaltet ist, um das vordere Ende des vorgebogenen Drahtes 26 in einen sich an die Eintrittsöffnung 40 anschließenden Schleusenkanal 42 zu führen. Im Schleusenkanal sind Dichtlippen 44 vorgesehen, die auf der Mantelfläche des vorgebogenen Drahtes 26 aufliegen, wenn der vorgebogene Draht 26 in den Schleusenkanal 42 eingeführt ist. Mittels der Dichtlippen 44 wird bei in den Schleusenkanal 42 eingeschobenem vorgebogenen Draht 26 eine Abdichtung hergestellt.

Figur 2 ist weiterhin zu entnehmen, daß der Schleusenkanal 42 im Bereich der Katheterspitze 32 von einer Membran 46 verschlossen ist, die beim ersten Vorschieben des vorgebogenen Drahtes 26 über die Katheterspitze 32 hinaus durchstochen wird.

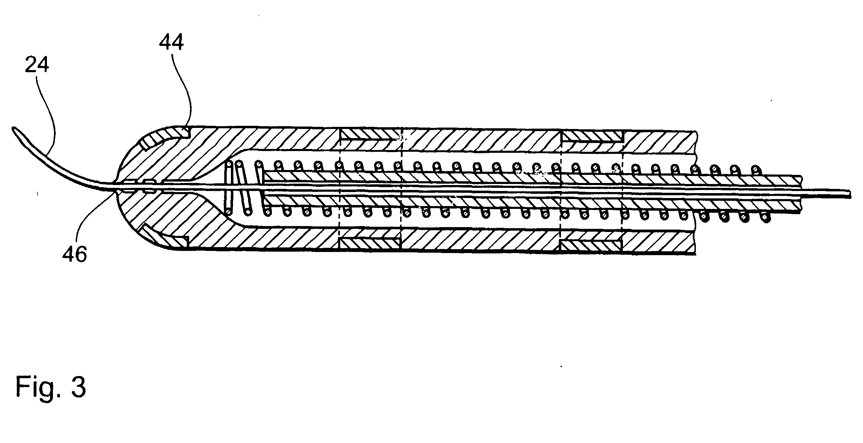

Dieser Zustand ist in Figur 3 dargestellt. Sobald der vorgebogene Draht 26 mit seinem distalen Ende 24 aus dem Katheter 10 austritt, ist er keinen Kräften mehr ausgesetzt, die ihn gegen seine Vorspannung strecken, so daß sich der vorgebogene Draht frei seiner Vorbiegung entsprechend krümmt. Die durchstoßene Membran 46 stellt außerdem eine weitere Dichtlippe dar, die zusammen mit den Dichtlippen 44 für eine Abdichtung des Katheterinneren gegenüber dem Katheteräußeren sorgen.

## Patentansprüche

1. Katheter zum Einführen in Blutgefäße, mit einem Lumen und darin angeordneten Führungsmitteln, wobei die Führungsmittel einen in Längsrichtung des Katheters (10) verschiebbaren vorgebogenen Draht (26) umfassen und wobei der Katheter (10) an seinem distalen Ende eine derartige Schleuse (38) aufweist, daß der vorgebogene Draht (26) durch die Schleuse (38) hindurch am distalen Ende aus dem Katheter (10) austreten kann, **dadurch gekennzeichnet, daß** die Schleuse (38) eine von dem vorgebogenen Draht (26) zu durchstoßende Membran (46) umfasst.

2. Katheter nach Anspruch 1, **dadurch gekennzeichnet, daß** der vorgebogene Draht (26) ein Federstahldraht ist.

3. Katheter nach Anspruch 1 oder 2, **dadurch gekennzeichnet, daß** der vorgebogene Draht (26) zumindest im Bereich seines distalen Endes (24) helixförmig vorgebogen ist.

4. Katheter nach Anspruch 1 oder 2, **dadurch gekennzeichnet, daß** der vorgebogene Draht (26) zumindest im Bereich seines distalen Endes (24) spiralförmig vorgebogen ist.

5. Katheter nach einem der Ansprüche 1 bis 4, **dadurch gekennzeichnet, daß** die Führungsmittel eine Führungshülse (22) für der vorgebogenen Draht (26) umfassen, die derart gestaltet ist, daß sie den vorgebogenen Draht (26) innerhalb des Katheters (10) in gestrecktem, vorgespanntem Zustand hält.

6. Katheter nach einem der Ansprüche 1 bis 5, **dadurch gekennzeichnet, daß** der vorgebogene Draht (26) relativ zum übrigen Katheter (10) um seine Längsachse drehbar ausgeführt ist mit am proximalen Ende des Katheters angeordneten Betätigungsmitteln (30) zum Drehen des vorgebogenen Drahtes (26) in Verbindung steht.

7. Katheter nach Anspruch 5, **gekennzeichnet durch** einen rohrartig ausgebildeten, als Führungshülse dienenden Mandrin (22) mit einem inneren Kanal (36), in dem der der vorgebogene Draht (26) geführt ist.

8. Katheter nach Anspruch 7, **dadurch gekennzeichnet, daß** der Mandrin (22) im Katheter längsverschiebbar und/oder um seine Längsachse drehbar ist und mit Betätigungsmitteln (28) zum Längsverschieben und/oder Drehen des Mandrins (22) in Verbindung steht.

9. Katheter nach einem der Ansprüche 1 bis 8, **dadurch gekennzeichnet, daß** die zu durchstoßende Membran aus elastischem Material derart ausgeführt ist, daß sie sich wieder schließt, wenn der vorgebogene Draht (20) wieder vollständig in den Katheter (10) zurückgezogen wird.

10. Katheter nach einem der Ansprüche 1 bis 9, **dadurch gekennzeichnet, daß** die Schleuse (38) auf ihrer dem Katheterinneren zugewandten Seite eine Eintrittsöffnung (40) für den vorgebogenen Draht aufweist, die trichterförmig gestaltet ist.

11. Katheter nach einem der Ansprüche 1 bis 10, **dadurch gekennzeichnet, daß** die Schleuse (38) eine Austrittsöffnung in der Außenseite des Katheters (10) aufweist, die sich in der Mitte einer gerundeten Katheterspitze (32) am distalen Ende des Katheters (10) befindet.

12. Katheter nach einem der Ansprüche 1 bis 11, **dadurch gekennzeichnet, daß** der Katheter (10) als Herzschrittmacher- oder Defibrillatorelektrode ausgeführt ist.

13. Katheter nach einem der Ansprüche 1 bis 12, **dadurch gekennzeichnet, daß** der vorgebogene Draht (26) Gold enthält.

14. Katheter nach Anspruch 13, **dadurch gekennzeichnet, daß** die Oberfläche des vorgebogenen Drahtes (26) zumindest in seinem vorderen, über die Katheterspitze (32) hinaus vorschiebbaren Ende von Gold gebildet ist.

## Claims

1. Catheter for introduction into blood vessels comprising a lumen and guide means arranged therein, the guide means including a pre-bent wire (26) displaceable in the longitudinal direction of the catheter (10), and the catheter (10) at its distal end comprising a lock (38) such that the pre-bent wire (26) can exit the catheter (10) at the distal end through the lock (38), **characterised in that** the lock (38) includes a diaphragm (46) to be pierced by the pre-bent wire (26).

2. Catheter according to claim 1, **characterised in that** the pre-bent wire (26) is a spring steel wire.

3. Catheter according to either claim 1 or claim 2, **characterised in that** the pre-bent wire (26) is pre-bent helically at least in the region of its distal end (24).

4. Catheter according to either claim 1 or claim 2, **characterised in that** the pre-bent wire (26) is pre-bent spirally at least in the region of its distal end (24).

5. Catheter according to any one of claims 1 to 4, **characterised in that** the guide means includes a guide sleeve (22) for the pre-bent wire (26), said guide sleeve being configured in such a way that it holds the pre-bent wire (26) in a straight pre-stressed state within the catheter (10).

6. Catheter according to any one of claims 1 to 5, **characterised in that** the pre-bent wire (26) is adapted to be rotatable relative to the rest of the catheter (10) about its longitudinal axis and is connected to actuating means (30), arranged at the proximal end of the catheter, for rotating the pre-bent wire (26).

7. Catheter according to claim 5, **characterised by** a bar (22) which is of a tubular configuration and which serves as the guide sleeve and which has an internal passage (36) in which the pre-bent wire (26) is guided.

8. Catheter according to claim 7, **characterised in that** the bar (22) is longitudinally slidable in the catheter and/or rotatable about its longitudinal axis and is connected to the actuating means (28) for longitudinal sliding movement and/or rotary movement of the bar (22).

9. Catheter according to any one of claims 1 to 8, **characterised in that** the diaphragm which is to be pierced is constructed from resilient material such that it closes again when the pre-bent wire (20) is completely withdrawn into the catheter (10) again.

10. Catheter according to any one of claims 1 to 9, **characterised in that** on its side towards the interior of the catheter the lock (38) has an entrance opening (40) for the pre-bent wire, the opening being of a funnel-shaped configuration.

11. Catheter according to any one of claims 1 to 10, **characterised in that** the lock (38) has an exit opening in the outside of the catheter (10), which is in the centre of the rounded catheter tip (32) at the distal end of the catheter (10).

12. Catheter according to any one of claims 1 to 11, **characterised in that** the catheter (10) is in the form of a cardiac pacemaker electrode or defibrillator electrode.

13. Catheter according to any one of claims 1 to 12, **characterised in that** the pre-bent wire (26) includes gold.

14. Catheter according to claim 13, **characterised in that** the surface of the pre-bent wire (26), at least in its front end which can be advanced beyond the catheter tip, is formed by gold.

## Revendications

1. Cathéter destiné à être introduit dans des vaisseaux sanguins, comportant un lumen et des moyens de guidage agencés dans celui-ci, les moyens de guidage comportant un fil pré-cambré (26) pouvant coulisser dans le sens longitudinal du cathéter (10) et le cathéter (10) comportant au niveau de son extrémité distale un passage (38) conçu de telle sorte que le fil pré-cambré (26) peut passer à travers le passage (38) et sortir du cathéter (10) par l'extrémité distale, **caractérisé en ce que** le passage (38) est muni d'une membrane (46) à transpercer par le fil pré-cambré (26).

2. Cathéter selon la revendication 1, **caractérisé en ce que** le fil pré-cambré (26) est un fil en acier à ressort.

3. Cathéter selon la revendication 1 ou 2, **caractérisé en ce que** le fil pré-cambré (26), au moins dans la zone de son extrémité distale (24), est pré-cambré en forme d'hélice.

4. Cathéter selon la revendication 1 ou 2, **caractérisé en ce que** le fil pré-cambré (26), au moins dans la zone de son extrémité distale (24), est pré-cambré en forme de spirale.

5. Cathéter selon l'une quelconque des revendications 1 à 4, **caractérisé en ce que** les moyens de guidage comportent une gaine de guidage (22) pour le fil pré-cambré (26), laquelle est conçue de telle sorte qu'elle maintient le fil pré-cambré (26) à l'intérieur du cathéter (10) dans une position redressée précontrainte.

6. Cathéter selon l'une quelconque des revendications 1 à 5, **caractérisé en ce que** le fil pré-cambré (26) est conçu rotatif par rapport au reste du cathéter (10) autour de son axe longitudinal et est relié à des moyens de manoeuvre (30), disposés au niveau de l'extrémité proximale du cathéter (10) et destinés à faire tourner le fil pré-cambré (26).

7. Cathéter selon la revendication 5, **caractérisé par** un mandrin (22) tubulaire formant la gaine de guidage et contenant un canal intérieur (36) dans lequel est logé le fil pré-cambré (26).

8. Cathéter selon la revendication 7, **caractérisé en ce que** le mandrin (22) est monté mobile longitudinalement dans le cathéter (10) et/ou rotatif autour de son axe longitudinal et est relié à des moyens de manoeuvre (28) destinés à déplacer longitudinalement et/ou à faire tourner le mandrin (22).

9. Cathéter selon l'une quelconque des revendications 1 à 8, **caractérisé en ce que** la membrane à transpercer est réalisée dans un matériau élastique, de telle sorte qu'elle se referme à nouveau lorsque le fil pré-cambré (26) est entièrement revenu dans le cathéter (10).

10. Cathéter selon l'une quelconque des revendications 1 à 9, **caractérisé en ce que** le passage (38), sur son côté orienté vers l'intérieur du cathéter, comporte un orifice d'entrée (40) pour le fil pré-cambré (26), lequel est conçu en forme d'entonnoir.

11. Cathéter selon l'une quelconque des revendications 1 à 10, **caractérisé en ce que** le passage (38) comporte un orifice de sortie dans la face extérieure du cathéter (10), lequel est disposé au centre d'un bout arrondi (32) du cathéter au niveau de l'extrémité distale du cathéter (10).

12. Cathéter selon l'une quelconque des revendications 1 à 11, **caractérisé en ce que** le cathéter (10) est conçu sous forme d'électrode de stimulateur cardiaque ou d'électrode de défibrillateur.

13. Cathéter selon l'une quelconque des revendications 1 à 12, **caractérisé en ce que** le fil pré-cambré (26) contient de l'or.

14. Cathéter selon la revendication 13, **caractérisé en ce que** la surface du fil pré-cambré (26), au moins dans son extrémité avant s'avançant au-delà de la pointe (32) du cathéter, est réalisée en or.
